# EUROPEAN PATENT SPECIFICATION

(11) **EP 4 054 486 B1**
(45) Date of publication and mention of the grant of the patent: **17.06.2026**
(21) Application number: 20838132.7
(22) Date of filing: 24.11.2020
(51) Int. Cl.: A61F 2/30, A61F 2/32, A61F 2/36

(54) **CERAMIC MONOBLOC FEMORAL COMPONENT, KIT AND SYSTEM COMPRISING THE SAME**
KERAMISCHE MONOBLOCK-OBERSCHENKELKOMPONENTE, KIT UND SYSTEM DAMIT
COMPOSANT FÉMORAL MONOBLOC EN CÉRAMIQUE, KIT ET SYSTÈME COMPRENANT CELUI-CI

(30) Priority: 28.11.2019 GB 201917377
(43) Date of publication of application: 14.09.2022
(73) Proprietor: MatOrtho Limited, Leatherhead Surrey KT22 7BA (GB)
(72) Inventor: DE VILLIERS, Danielle, Reigate RH2 7AB (GB); COLLINS, Simon Nicholas, Tetbury GL8 8NT (GB); CULLUM, Charles Jonas Ambrose, Worcester, WR2 4ET (GB); TUKE, Michael Antony, Guildford Surrey GU1 3TF (GB); KIRWAN, David Phillip, Albury, New South Wales 2640 (AU)
(74) Representative: Hocking, Adrian Niall
(86) International application number: PCT/GB2020/052984
(87) International publication number: WO 2021/105657

(56) References cited:
- EP-A2- 1 138 283
- GB-A- 2 554 844
- US-A1- 2002 010 070
- US-A1- 2005 187 638

## Description

The present invention relates to a hip prosthesis system, and more particularly to the femoral component thereof. The present invention also relates to a kit comprising a plurality of different dimensioned femoral components for use in a hip replacement. The present invention also relates to a system comprising a femoral component and an acetabular cup component of a hip prosthesis system.

The hip is a ball and socket joint which allows the femur to rotate and be movable from front to back and side to side. With time, the hip joint, and in particular the cartilage between the articular surfaces of the acetabulum and femur, deteriorates due to wear and tear and degenerative medical conditions such as arthritis. This typically leads to a reduction in mobility and an increase in pain. Hip replacements or arthroplasty are therefore becoming more prevalent, particularly in countries with an ageing population. In the UK alone, over 90,000 hip replacements are carried out each year.

A hip prosthesis typically comprises an acetabular cup and a femoral component. The femoral component has a femoral ball head, which fits within the cup, and a stem, receivable within the femur. In modular femoral components, the head and stem are usually connectable together via taper locking. However, wear and tear of the modular femoral component, particularly at the tapers, may produce debris leading to 'trunnionosis' which is accelerated wear of the tapers, causing micro-movements and taper mismatch. This can lead to pain, lysis of surrounding cells, loosening, and potentially joint failure and need for hip revision.

Furthermore, femoral components are currently at least partly formed of metal, such as stainless steel, titanium alloys, or cobalt Chrome molybdenum alloys. Such metallic components may release particles and metal ions, such as titanium, cobalt and Chromium, particularly at the tapers. These particles and metal ions have been linked to potential allergic reactions, soft tissue necrosis, aneuploidy, pseudotumours, carcinogenesis or other adverse health effects for the patient.

GB2554844A (D1) relates to a ceramic femoral resurfacing head prosthesis having a convex outer surface, a concave inner surface and a ceramic stem. The stem has at least at two-part angular transition in a longitudinal direction of the stem.

US 2002/0010070A1 (D2) discloses a biomedical metal femoral component comprising zirconia toughened alumina ZTA.

The present invention seeks to provide a solution to these problems.

According to a first aspect of the present invention, there is provided a ceramic monobloc femoral component as defined in claim 1.

The femoral component is formed of ceramic such that the amount of metal required is substantially reduced, minimised or eliminated. Ceramic material provides high strength whilst being bio-inert or highly biocompatible. Thus, the side-effects associated with metal femoral implants are absent or their likelihood is reduced. Ceramic materials have very low wear rates and removal of the tapers by providing a unitarily or integrally formed femoral component reduces the likelihood of debris and trunnionosis.

Beneficially, the ceramic femoral stem may further comprise a collar terminating between a tangential plane of an equatorial circumference of the ceramic head and a polar-axial plane parallel to said tangential plane. The collar provides additional stability to the femoral component, particularly when abutting the femur.

Furthermore, the head may be retroverted relative to the sagittal plane of the ceramic monobloc femoral component. Such asymmetry may assist in correct restoration of the pre-surgical anatomy leading to improved joint function and reduced impingement/dislocation thereafter.

Furthermore, the percentage of Aluminium Oxide may be or may substantially be 70% to 85% by weight. Alternatively, the percentage of Aluminium Oxide may be or may substantially be 15% to 25% by weight. Furthermore, the percentage of Zirconium Oxide may be or may substantially be 15% to 25% by weight. Alternatively, the percentage of Zirconium Oxide may be or may substantially be 70% to 85% by weight. Each ceramic material, whether used alone or together as an Aluminium Oxide/Zirconium Oxide composite is bio-compatible whilst having desirable properties such as suitable fracture toughness and/or small grain size. The values of the desirable properties are determined by, and consequently, alterable by changing the exact composition of the ceramic material.

Preferably, the Zirconium Oxide is toughened Zirconium Oxide. Furthermore, the Zirconium Oxide may be toughened by Strontium Oxide. Alternatively, the Zirconium Oxide may be toughened by Yttrium Oxide. The toughened Zirconium Oxide is stabilised in a tetragonal structure, which upon transitioning to a monoclinic structure, expands in volume. A crack in the femoral component may cause the transition to occur, such that the expanding Zirconium Oxide substantially halts or reduces the propagation or further propagation of the crack, thereby enhancing the fracture toughness of the material. Non-toughened Zirconium Oxide may be used, however.

Optionally, the ceramic head may further comprise a flange having a medial extent which may be equivalent to or greater than half a radius of a neck of the ceramic femoral stem.

Furthermore, the ceramic femoral stem may further comprise a shoulder terminating between a tangential plane of an equatorial circumference of the ceramic head and a polar-axial plane parallel to said tangential plane. This enables a snug fit within a cavity of the femur whilst positioning the head at an appropriate angle, in-use, relative to the acetabulum. Additionally, the shoulder or the outermost tip thereof provides a clear reference point for the surgeon during surgery, by indicating how deep the femoral implant is to be received within a femur.

According to a second aspect of the present invention, there is provided a ceramic monobloc femoral component kit comprising a plurality of ceramic monobloc femoral components in accordance with the first aspect of the invention, a first said ceramic monobloc femoral component having a said femoral stem and a said femoral head, and a second said femoral component having a said femoral stem and a said femoral head, wherein the said femoral stems are same-dimensioned and the femoral heads are different-dimensioned. A plurality of femoral components having different sized heads, but similarly sized stems enables a larger head to be used, as required, without requiring removal of a larger volume of femoral bone. In other words, a range of diametrically different femoral components may be used with a same-sized cavity in the femur, requiring less surgical intervention to adjust the cavity volume.

According to a third aspect of the invention, there is provided a total hip replacement system comprising at least one ceramic monobloc femoral component in accordance with the first aspect of the invention, and at least one ceramic acetabular cup component for receiving the ceramic head of the ceramic monobloc femoral component. This hip joint, provided as a system or kit of parts, may be used in surgery and does not comprise any components susceptible of releasing metal ions.

Advantageously, the total hip replacement system may comprise a plurality of ceramic monobloc femoral components and a plurality of ceramic acetabular cup components, each said ceramic monobloc femoral component being matched with at least a pair of dimensionally different said ceramic acetabular cup components. Furthermore, a said ceramic monobloc femoral component may be matched with at least three dimensionally different said ceramic acetabular cup components. The inventory stocked by the hospital is reduced, which in turn reduces storage space and costs.

Beneficially, at least one of the femoral component and the acetabular cup may be or may substantially be free from metal other than in oxide, carbide or nitride forms. This removes a source of harmful metal ions.

Also disclosed is a method of manufacturing a ceramic monobloc femoral component or cup component of a hip prosthesis, the method comprising the steps of: a] providing a mould shaped to form the monobloc femoral component and/or cup component; b] providing, preferably toughened, Zirconium Oxide in a powdered form; c] inserting the powder into the mould; d] processing the powder into the green compact; and e] sintering the green compact. This provides an easy, reliable manufacturing process of a complex part. The steps a] and b] may be performed in any order. Although toughened Zirconium Oxide is preferred, non-toughened Zirconium Oxide may be used.

Preferably, in step a] the Zirconium Oxide is toughened with at least one of: Yttrium Oxide, Strontium Oxide, Strontium Aluminate, and Chromium Oxide. Furthermore, the method may comprise a further step of providing Aluminium Oxide in powdered form, and mixing the Aluminium Oxide with the toughened Zirconium Oxide to form a powder mixture. Preferably, this step occurs prior to step c], to ensure the mixture of Aluminium Oxide and Zirconium Oxide is or is substantially homogenous prior to insertion into the mould. This step may also occur after step b], but it could easily be envisioned that Aluminium Oxide may be provided first, and toughened and/or non-toughened Zirconium Oxide may be provided secondarily and mixed with the Aluminium Oxide.

Optionally, the Aluminium Oxide may be provided in the range of or substantially of 15% to 85% by weight and the toughened Zirconium Oxide may be provided in the range of or substantially of 15% to 85% by weight. Furthermore, the Aluminium Oxide may be provided as or approximately as 20% by weight and the toughened Zirconium Oxide may be provided as or approximately as 80% by weight. Alternatively, the Aluminium Oxide may be provided as or approximately as 80% by weight and the toughened Zirconium Oxide may be provided as or approximately as 20% by weight. The properties of the product may be altered by altering the composition of the ceramic material.

Also disclosed is a method of use of the femoral implant, the method comprising the steps of: a] selecting a ceramic monobloc femoral component, preferably in accordance with the first aspect of the invention; b] inserting the body of the femoral component into a prepared femur cavity; and c] inserting the head of the femoral component into an acetabulum socket or an acetabular cup engaged with the acetabulum.

The invention will now be more particularly described, by way of example only, with reference to the accompanying drawings, in which:
Figure 1 shows a side view of an embodiment of a ceramic monobloc femoral component, in accordance with the first aspect of the invention;
Figure 2 shows a back view of the femoral component of Figure 1, in accordance with the first aspect of the invention;
Figure 3 shows another side view of the femoral component of Figure 1, in accordance with the first aspect of the invention;
Figure 4 shows a front view of the femoral component of Figure 1, in accordance with the first aspect of the invention;
Figure 4A shows a side view of the femoral component of Figure 3 with engagement-enhancing portions omitted for clarity, outlining a shoulder and a collar of the femoral component;
Figure 4B shows a cross-sectional view along the line A-A' of the femoral component of Figure 4A;
Figure 5 shows an exploded view of an embodiment of a total hip replacement system, in accordance with the third aspect of the invention;
Figure 6 shows a side view of a second embodiment of a ceramic monobloc femoral component, in accordance with the first aspect of the invention;
Figure 7 shows a cross-sectional view of a third embodiment of the femoral component, in accordance with the first aspect of the invention;
Figure 8A shows a side view of a fourth embodiment of the femoral component, in accordance with the first aspect of the invention;
Figure 8B shows a cross-sectional view along the line B-B' of the femoral component of Figure 8A;
Figure 9A shows a side view of a fifth embodiment of the femoral component, in accordance with the first aspect of the invention;
Figure 9B shows a cross-sectional view along the line C-C' of the femoral component of Figure 9A;
Figure 10A shows a side view of a sixth embodiment of the femoral component, in accordance with the first aspect of the invention; and
Figure 10B shows a cross-sectional view along the line D-D' of the femoral component of Figure 10A.

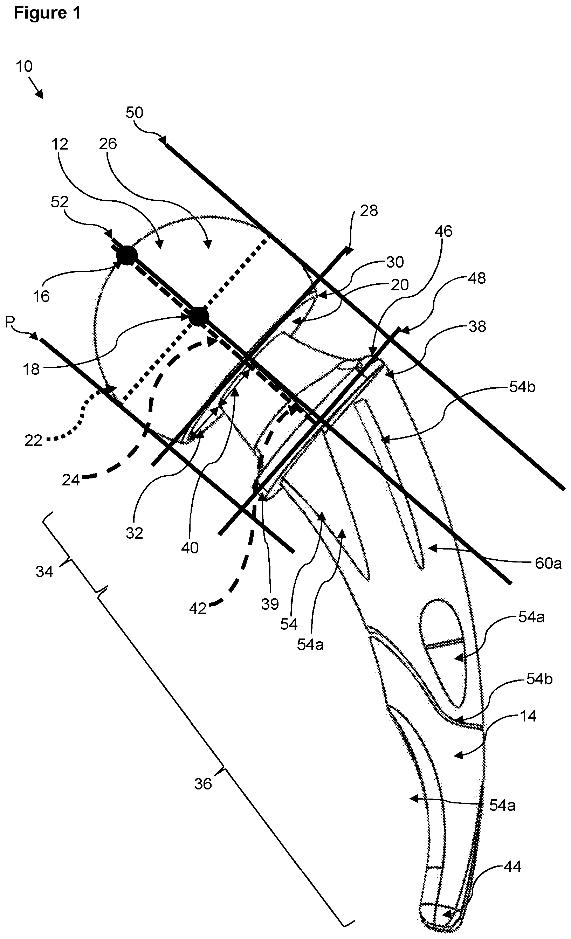

Referring firstly to Figure 1, there is shown a femoral component generally indicated as 10 for a hip replacement prosthesis.

The femoral component or leg component 10 is a prosthetic replacement of at least the natural femur head, and here, both femur head and neck. To improve the engagement and/or inhibit or prevent rotation of the femoral component 10 relative to the femur of the patient, the femoral component 10 is preferably asymmetric such that it is designed to be engageable with only one of the left and the right legs. In the shown embodiment, the femoral implant is for a left leg.

Importantly, the femoral component 10 is formed of a ceramic material. Said ceramic material comprises Aluminium Oxide and Zirconium Oxide. In this embodiment, the ceramic material is preferably an Aluminium Oxide and Zirconium Oxide composite. The composite is preferably formed as a homogenous mixture. A ceramic material comprising Aluminium Oxide and Zirconium Oxide may be referred to as Zirconia Toughened Alumina ZTA or Alumina-toughened Zirconia ATZ, depending on the relative proportions of Zirconium Oxide to Aluminium Oxide.

The ceramic material includes a percentage of Aluminium Oxide, also known as Alumina, in a range of or substantially of 15% to 85%, or any value therebetween, by weight. An alternative range may be or substantially be 15% to 25% w/w. Preferably, the Aluminium Oxide is provided within the range of or substantially of 70% to 85% w/w and more preferably as or approximately as 80% w/w and/or substantially 82% v/v. The Aluminium Oxide in the present embodiment is preferably provided as 81.6% v/v.

In addition to Alumina, the ceramic material includes a percentage of Zirconium Oxide, also known as Zirconia, in a range of or substantially of 15% to 85%, or any value therebetween, by weight. An alternative range may be or substantially be 70% to 85% w/w. Preferably, the Zirconium Oxide is provided within the range of or approximately of 15% to 30% w/w or even 15% w/w to 25% w/w. More preferably, the Zirconia is provided as or substantially as 20% w/w and most preferably as or substantially as 17% w/w or v/v.

Zirconium Oxide adopts a stable monoclinic crystal structure at room temperature. At about 1173°C to 1175°C, Zirconium Oxide transitions to a tetragonal structure, and transitions again at about 2370°C to a cubic structure.

Zirconium Oxide can be at least partially stabilised in the tetragonal and/or cubic phases when blended with one or more other Oxides. Zirconium Oxide is said to be stabilised, doped or toughened. In this embodiment, the Zirconium Oxide is toughened or stabilised by Strontium Oxide, although any other dopant or combination of dopants may be envisioned. The percentage of Strontium Oxide by weight may be between 0.1% to 10% w/w, such as approximately 3% w/w.

As sintering Strontium Oxide forms Strontium Aluminate, the ceramic material may comprise Strontium Aluminate instead of or in addition to Strontium Oxide. The ceramic material also preferably comprises Chromium Oxide. An example of such a ceramic material may comprise or approximately comprise 82% v/v Alumina; approximately 17% v/v Zirconia particles, preferably tetragonal; approximately 0.5% v/v Strontium Aluminate and approximately 0.5% v/v Chromium Oxide. A suitable material may be BIOLOX (RTM) delta.

If the toughened Zirconium Oxide is in the metastable tetragonal phase and a crack or fracture forms in the ceramic material, this may be sufficient to cause the Zirconium Oxide to transition from a tetragonal structure to the monoclinic structure. The transition from the tetragonal structure to the monoclinic structure involves an increase in volume of the Zirconium Oxide, typically 3% to 5%, which advantageously inhibits or limits the further propagation and/or partially closes, reduces or redirects the crack in the Zirconium Oxide.

The Aluminium Oxide / toughened Zirconium Oxide composite may have a theoretical density in the range of 3 g/cm³ to 9 g/cm³ or any value therebetween. More preferably, the theoretical density of the composite is 4 g/cm³ to 6 g/cm³. In this embodiment, the density is substantially 4.37 g/cm³. The fracture toughness is the capacity of a material to resist crack propagation. Preferably the ceramic material may have a fracture toughness equal to at least 4.5 MPa√m and more preferably, of at least 6.5 MPa√m. Here, the fracture toughness is at least 6.5 MPa√m. A reduced grain size enables a reduction in the surface roughness, particularly after polishing such that the wear rate and/or coefficient of friction are low. This further reduces the likelihood of debris formation. In this embodiment, the grain size may be or approximately be in the range of 0.1 µm to 1.8 µm or any value therebetween. More preferably, the grain size is 0.3 µm to 0.9 µm, or even 0.2 µm to 0.8 µm. In this embodiment, the grain size is or is substantially 0.6 µm, and more specifically 0.56 µm. The composite may also have a biaxial bending strength of at least 500 MPa, and more preferably of at least 800 MPa. The hardness of the ceramic, relating to the resistance of the material to deformation and wear resistance, is preferably at least 14 GPa, and more preferably is substantially 19 GPa. Most preferably, the hardness is 19.61GPa or 2000HV. Such a composite provides a high fracture strength, high resistance to ageing and has a lower likelihood of forming debris.

The femoral component 10 has a femoral head 12 and a femoral stem 14. The femoral component 10 is a one-piece such that the femoral head 12 and the stem 14 are not releasably connectable with each other. In other words, the femoral component 10 may be said to be monobloc, unitarily or integrally formed, and non-modular.

The head 12 is or is substantially spherical and is typically a segment or portion of a sphere slightly greater than a hemisphere. In other words, the head 12 may be considered as resulting from the partial truncation, frustum or spherical segment of a fully spherical head. The head 12 has a pole 16; a centre 18; a flange 20; an equatorial circumference 22, shown as a dotted line in Figure 1, centred around the centre 18; and an axis 24, represented as a dashed line in Figure 1, traversing the centre 18 and the pole 16 of the head 12. As such, the axis 24 may be referred to as a polar axis.

The head 12 has a diameter which may be within the range of 32 mm to 64 mm, although a diameter outside of this range may be envisioned. More preferably, the femoral head 12 has a diameter between 32mm and 48mm.

The head 12 defines a zone or part-spherical surface 26, part of which is engageable with or at least receivable by or in an acetabular cup. The part-spherical surface 26 may be referred to as an articular surface, an articulating surface, an articulation surface or a convex outer contact surface. The part-spherical surface 26 is preferably continuous. The head 12 is preferably solid, for structural integrity and ease of manufacture, although a partially or fully hollow head may alternatively be envisioned, for example, to reduce the quantity of material required for weight and cost considerations.

The flange 20 is a rim or ledge extending around at least part of, and here the whole circumference of the sphere. The flange 20 may also be considered to be an overhang, lip or shoulder. The flange 20 defines a planar under surface 28, the plane of the under surface 28 being shown in cross-section in Figure 1 as a line. The flange 20 is or is substantially flat or planar and said plane preferably is or is substantially normal to the polar axis. The flange 20 is contiguous with the part-spherical surface 26, and as such, forms an edge 30 with the part-spherical surface 26. Said edge 30 preferably defines an unbroken or continuous arc, such as a circle or ellipse. The flange 20 has a radial, lateral or medial extent 32 and is preferably centred around the polar axis.

The femoral stem 14 is an elongate portion or projection which is engageable with the leg. In particular, the stem 14 is at least partly receivable within a cavity in the femur of a patient, preferably via an interference fit. The femoral stem 14 is provided as a size from a range of, typically, ten sizes. As the femoral stem 14 in this embodiment is asymmetric, there are typically at least twenty different stem configurations or dimensions due to each size having a left or right configuration. In the shown embodiment, the femoral stem 14 is a short stem or mini stem, and comprises a neck 34 and a body 36. The femoral stem 14 also comprises in this case a shoulder 38 and a collar 39, although either or both of these features may be optional.

The neck 34 connects the body 36 of the stem 14 and the femoral head 12. The neck 34 is preferably integrally formed with the head 12, and specifically meets, joins, merges into, or is contiguous with the planar under surface 28. The neck 34 is a narrowing in the stem 14 to facilitate and/or increase the range of angles at which the head 12 and an acetabular cup engage. The neck 34 meets the planar under surface 28 and/or flange 20 and is at least partially surrounded by the flange 20. Preferably, the cross-section of the neck 34 is substantially circular in cross-section where it meets the planar under surface 28 such that the neck 34 has a radius 40. The circular neck 34 meets the circular planar under surface 28 substantially centrally such that the radial, lateral and/or medial extent 32 of the flange 20 is substantially identical at any point around the head 12. At or adjacent to where the neck 34 and the planar under surface 28 meet, the radial, lateral and/or medial extent 32 of the flange 20 is preferably equivalent to or greater than half the radius 40 of the neck 34 i.e. at least a quarter of the diameter of the neck 34. This provides a relatively large head circumference 22 with a proportionately thinner stem 14 and/or neck 34. It could however, easily be envisioned that the flange extent may be less than half the diameter, and/or less than half the radius of the neck, for structural integrity of the neck.

Preferably, the neck 34 transitions smoothly and/or arcuately into the planar under surface 28 and/or flange 20 as shown, preferably without forming an edge 30. This reduces the likelihood of structural failure between the head 12 and the neck 34.

Although the neck may be cylindrical, preferably the neck 34 tapers outwards, substantially smoothly, from being circular in cross-section at or adjacent to the planar under surface 28 and/or flange 20 to substantially tear-shaped, egg-shaped, elliptical or ovoidal, or circular with an increasing radius in cross-section with increasing distance from the head 12. However, the centres at various cross-sections form a substantially linear central axis, which may be referred to as a neck axis 42, represented as a dashed line in Figure 1. Said neck axis 42 is preferably colinear and/or aligned with, or at least parallel to the polar axis 24 but this need not necessarily be the case.

Importantly, the neck 34 is integrally formed with the body 36. Equally importantly, the neck 34 is also preferably integrally formed with the head 12. This removes the need for the head 12 and the neck 34 and/or the neck 34 and the body 36 to have complementarily-engageable tapers. The absence of the detachably engageable tapers prevents or reduces the likelihood of debris forming where the tapers would contact each other. Thus, the head 12 merges or substantially merges with the stem 14.

The body 36 of the stem 14 engages with a cavity of the femur, which is here at least the medullary cavity, medullary canal, or marrow cavity. Thus, the femoral component 10 may be said to be at least partly intramedullary. The neck 34 meets, merges, substantially merges or transitions into the body 36. Whilst the body 36 is elongate, preferably the femoral component or implant 10 has a short-stem and/or mini stem. Preparation of the femur in anticipation of insertion of the stem 14 involves bone removal, in particular, the head and neck of the natural bone and, optionally, hollowing the shaft to widen the cavity. This hollowing however, weakens the remaining natural bone and alters the forces and/or stresses distribution. A short-stem 14 advantageously results in more of the natural femur being conserved. Furthermore, the resulting force and/or stress distribution approximates more closely that observed in natural, unresected femurs. In turn, the risk of structural failure is reduced.

Preferably the body 36 is also at least partly curved as shown. The body 36 in this embodiment is curved in at least two directions or axes. This inhibits or prevents undesired movement, particularly rotation within the femur. The curvature or double curvature may also accommodate the curvature of the cavity and/or reduce or avoid compressing the bone at or adjacent to the proximal end of the femur shaft.

The curvature may also improve the distribution of forces within the femur. The femur neck anteversion is an angle measuring the relative positioning of the femoral head and neck, relative to the femoral trochanters, when viewed from above or from a superior view. Said angle may be 15°, in which case, the femur has neutral anteversion. Alternatively, the angle may be or substantially be below 15°, corresponding to a retroverted femur; or, more commonly, the angle may be greater than 15°, such that the femur is anteverted. Anteversion is characterised by the stem 14 contacting the cavity walls or endo-cortical surfaces of the femur, along the posterior wall, the anterior wall and the posterior wall, in sequence with increasing distance from the femoral component head. In retroverted femora, the stem contacts the anterior wall, posterior wall and anterior wall in sequence with increasing distance from the head. In neutral femora, the stem aligns better with the cavity such that there are no three contact points. The points of contact between the stem and the bone result in forces and/or stress, for example compression, being applied to the femur, particularly at a single location or small surface area, which may be damaging and weakening the structural integrity. Here, the stem 14 curvature better follows the anatomy of retroverted or, in particular, anteverted femoras, such that the contact points are removed and/or contact occurs over a larger surface, thereby distributing the forces. Additionally or alternatively, having multiple contact points and/or surfaces may prevent or inhibit rotation of the stem 14 within the femur.

The body 36 also has a decreasing taper, with increasing distance from the head 12 and ends with a distal tip 44. In other words, the cross-sectional area of the body 36 decreases with increasing distance from the head 12. In the present embodiment, the body 36 has a double taper as it narrows with increasing distance from the head 12 in two dimensions. The cross-section of the body 36 and/or the neck 34 is substantially elliptical, although other cross-sections may be envisioned such as circular, lachrymiform or tear-shaped, oval or egg-shaped, or any other at least partly curved and/or polygonal cross-section, such as square, hexagonal, or octagonal.

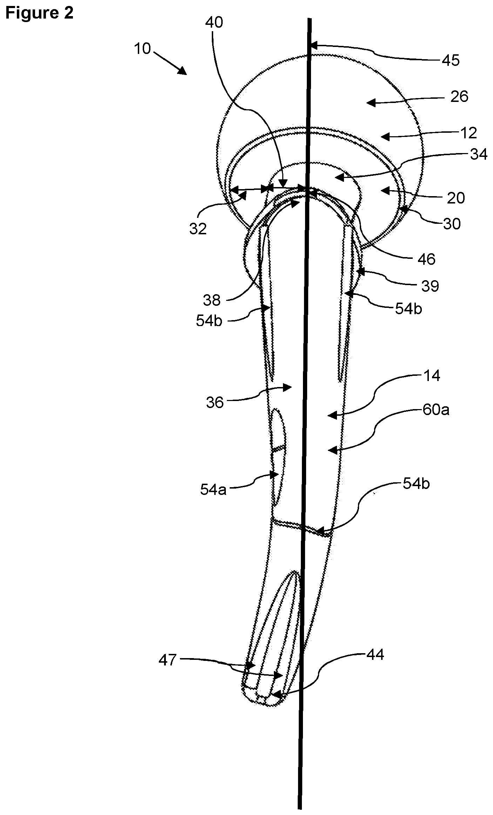

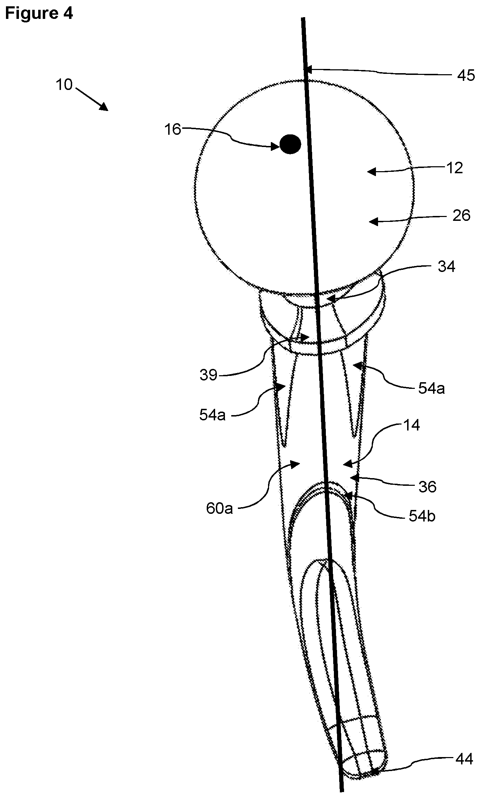

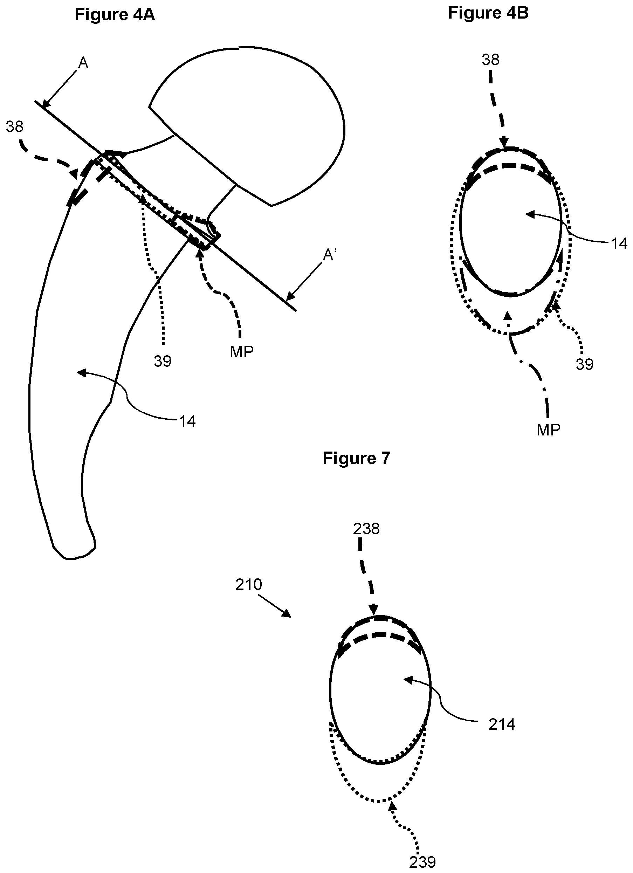

The distal tip may be in alignment with the rest of the body and/or in a sagittal plane thereof, but preferably, here, the distal tip 44 is out of alignment and/or out of the sagittal plane 45, represented in cross-section in Figures 2 and 4 as a line. Preferably, the longitudinal extent of the distal tip 44 extends at an angle relative to the sagittal plane 45 and/or the body axis. Said angle is preferably in the range of 2 to 30°, and more preferably, between 10° and 20°. Most preferably, the angle is or is substantially 15°. This feature further inhibits rotation of the stem relative to the femur. The distal tip 44 is preferably rounded. Furthermore, a bone-contacting side of the distal tip 44 may be rounded, flattened or may have ridges and/or dimples. In the present embodiment, the implant has two ridges 47 as shown, but any number of ridges may be envisioned. As such, the implant may be considered to have a twin tip. The groove formed therebetween may be of any shape, such as planar or at least partly curved.

The head 12 is traversed by the sagittal plane 45. Preferably in this embodiment, the head 12 is not centrally traversed by said plane 45 as best shown in Figure 4, such that the head 12 and/or the pole 16 and/or centre 18 is/are offset from or out of the sagittal plane 45. Such asymmetry may assist in correct restoration of the pre-surgical anatomy leading to improved joint function and reduced impingement/dislocation thereafter. Preferably, the polar axis 24 and/or neck axis 42 forms an angle with the body axis and/or the sagittal plane 45, said angle being within the range of 4° to 20°, more preferably within the range of 6° to 10°. Most preferably, the angle is or is substantially 8°. As best illustrated in Figure 4, the head 12 is preferably retroverted. However, it may be envisioned that the head may be anteverted. Alternatively, the head may be symmetric about the sagittal plane such that the head and/or the pole and/or centre of the head may be in the sagittal plane.

The shoulder 38 is defined as a bulge, prominence or protrusion which is, in this embodiment, integrally formed with the neck 34 and/or the body 36. The shoulder 38 is more clearly indicated as the portion in dashed lines in Figure 4A and in Figure 4B. The shoulder 38 enables a snug fit within the cavity whilst adjusting the angle of the neck 34 and consequently femoral head 12 relative to the acetabulum and/or femur. In the shown embodiment, the shoulder 38 is positioned at or adjacent to where the neck 34 and the body 36 merge. As the neck 34 has increasing taper and the body 36 decreasing taper with increasing distance from the head 12, the stem 14 is thickest or widest at the junction between the neck 34 and body 36 in latitudinal cross-section. This is due to the tapering of the neck 34 and body 36 but also in this case, to the presence of the shoulder 38.

An outermost tip, distal tip or a point 46 of the shoulder 38 may, in-use, be level with or contained within a plane 48, represented in cross-section as a solid line in Figure 1, defining the end of the patient's femur following resection of the bone. Thus, the shoulder 38 also provides an indication, reference point or marker to enable more accurate positioning, in particular depth of insertion of the femoral component 10 relative to the resected bone. An increased contact surface enables an improved engagement with the femur and a more preferable distribution of forces, thereby reducing the risk of failure of the implant 10. The body 36 also comprises a body axis (not shown) which is defined as comprising the substantially central point of each cross-section along the longitudinal extent of the body 36. In this embodiment, the body axis is curved, and having curvature along two axes.

The outermost tip 46 of the shoulder 38 is positioned between a plane 50, shown in cross-section as a solid line in Figure 1, tangential to the equatorial circumference 22 of the head 12, and a polar-axial plane 52, shown in cross-section as a solid line in Figure 1. The polar-axial plane 52 is defined as a plane parallel to the tangential plane 50 and containing or at least is parallel with the polar axis 24 and/or neck axis 42. The outermost tip may even be contained within either plane. Thus, the shoulder 38, and particularly the outermost tip 46 thereof, terminates between the tangential plane 50 of the equatorial circumference 22 of the head 12 and the polar-axial plane 52, parallel to said tangential plane 50 and comprising or parallel to the polar axis 24.

The collar 39, is defined as a bulge, prominence or protrusion which is, in this embodiment, integrally formed with the neck 34 and/or the body 36. Thus, this femoral component 10 may be said to be "collared". Said collar 39 may also be referred to as an overhang, extension, nub or projection or protrusion. The collar 39 extends away from the stem, preferably but not necessarily perpendicularly or substantially perpendicularly to the polar axis 24 and/or neck axis 42. In Figures 4A and 4B, the collar 39, indicated as dotted lines, or at least a major portion MP, volume or surface thereof, indicated as a dot-dashed line, is positioned opposite or substantially opposite the shoulder 38, although it could be envisioned that the collar may extend in any or all directions from the stem. The collar may be symmetrically positioned around the stem, but preferably is or is substantially asymmetric around the stem 14.

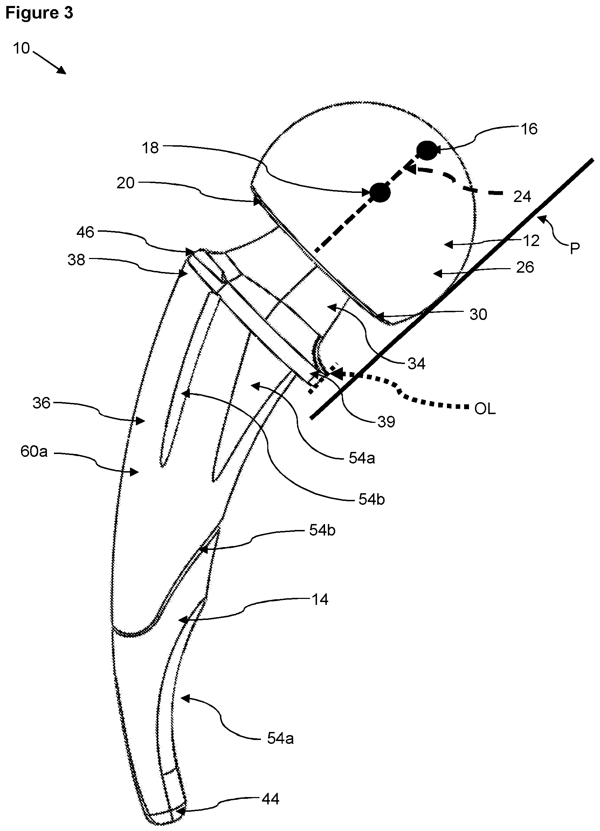

Similarly to the shoulder 38, the collar 39 may have an outermost tip, distal tip, point, surface or in the present embodiment, an outermost line OL, indicated as a dotted line, as best seen in Figure 3. Said outermost line OL may, in-use, be contained within or intersect, preferably perpendicularly, with the plane 48 containing the outermost tip 46 of the shoulder 38.

Furthermore, the outermost line OL of the collar 39 is positioned at or between a further plane P, tangential to the equatorial circumference 22 of the head 12, and a or the polar-axial plane 52, similarly to the shoulder 38, although it could be envisioned that part of the collar may extend beyond a tangential plane of the equatorial circumference. Preferably, said further plane is or is substantially parallel to the plane 50 and/or to the polar-axial plane 52, although this may not be necessarily the case.

As shown, the collar 39 extends around or at least partly around the stem and preferably meets the shoulder 38. It could easily be envisioned however that the collar does not meet or overlap with the shoulder.

The collar 39 is preferably part of the neck 34 as shown, although it may be envisioned that the collar may be formed as part of either or both the neck and the body, and/or be detachably connectable from either. The collar 39 improves the engagement with the bone and consequently the stability of the femoral component 10. The stem 14 further comprises at least one engagement-enhancing portion 54 to improve or enhance the engagement of the cavity and the femoral component 10 via interference fit, although the or any number of the at least one engagement-enhancing portion may be omitted. The at least one engagement-enhancing portion 54 may prevent or inhibit undesirable motion relative to the femur, such as rotation. As such, the at least one engagement-enhancing portion 54 may be referred to as an anti-rotation feature. Said engagement-enhancing portion 54 may be at least one of a protrusion, a lip, a ridge, a depression, a dimple, a facet, a recessed region, a flattened portion, a chamfered portion, a recess or groove.

A second function of the at least one engagement-enhancing portion 54 may be to encourage bone growth therein, thereagainst, or therearound, to further improve the engagement of the stem 14 and the femur.

A further function of the at least one engagement-enhancing portion 54 may be to alter the distribution of forces throughout the femoral implant by altering the cross-section of the stem 14. The engagement-enhancing portions 54 may alter the cross-section of the neck 34 and/or body 36. In the shown embodiment, there are seven engagement-enhancing portions 54 although any number may be envisioned. Four of the seven are substantially recessed regions, indicated as 54a.

Preferably, all the recessed regions 54 extend solely on or are positioned solely on the body 36. However, it could be envisioned that at least one, and any number of recessed regions may extend at least in part along the neck. Two of the recessed regions 54a are or are substantially symmetrical to each other, preferably via sagittal plane symmetry as shown in Figure 4, but asymmetry in shape and/or position may be envisioned. For example, the two recessed regions may be level with or offset along the sagittal plane relative to each other. Preferably, the two said recessed regions 54a are or are substantially triangular, but circular, oval, rectangular or any other polygonal, and/or curved shape may be envisioned.

A further of the four said recessed regions 54a is positioned at or around the mid-shaft antero-lateral side of the body 36, as best shown in Figure 2. Said recessed region 54a may be formed as two sub-regions and/or have a bar or separation therealong, as shown, but this need not necessarily be the case. The fourth of the four said recessed regions 54a is positioned at or about the distal tip 44.

The other three engagement-enhancing portions 54 are or are substantially grooves, and, to distinguish them from the recessed regions 54a, are indicated as 54b. The grooved engagement-enhancing portions 54b are located in or on the body 36. Preferably at least one of the engagement-enhancing portions 54b also extends into or onto the neck 34, but this feature may be omitted.

The femoral component 10 may be provided as part of a kit comprising a plurality of femoral components 10. Preferably, two or more of the ceramic monobloc femoral components 10 of the kit have a same-dimensioned ceramic femoral stem 14 and a different-dimensioned ceramic head 12. For example, the kit may comprise two femoral components 10, both having a Size Five stem 14 but one may have a head 12 with a diameter of 40 mm, and the other a head 12 of 44 mm in diameter.

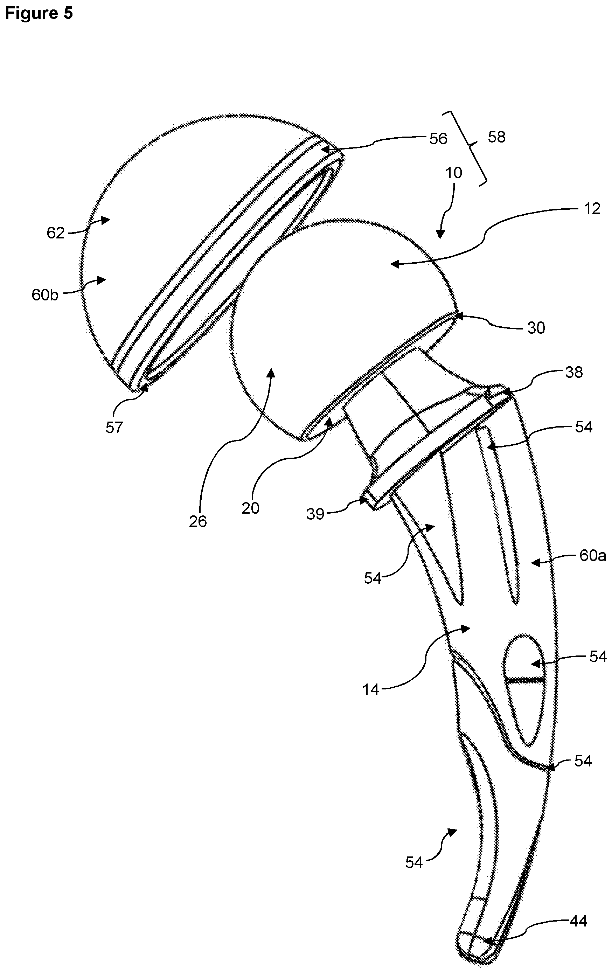

As shown in Figure 5, the femoral component 10 or the kit of femoral components 10 may be provided with at least one acetabular cup component 56 for receiving the ceramic head 12 of the ceramic monobloc femoral component 10, as part of a total hip replacement system 58. The cup 56 or part thereof is formed of ceramic. As such, at least one of the femoral component 10 and the acetabular cup 56, and preferably both, are metal-free, so as to remove a source of metal ions. The acetabular cup 56 may have an outer and/or inner diameter, measured at or adjacent to a rim 57 of the cup 56. Said inner cup diameter is typically within the range of 32 mm to 64 mm, although a cup with any diameter outside of this range may be envisioned and/or these dimensions may be of the outer diameter.

At least one of the femoral components 10 in such a total hip replacement system 58 may be matched with at least a pair of dimensionally different said ceramic acetabular cup components 56. This increased versatility reduces the size of the inventory that a hospital is required to source and stock.

Preferably, the same dimensioned femoral component 10 may be usable with at least two, more preferably at least three cups of different sizes. In the present embodiment, a femoral component 10 may be used with up to five dimensionally different said ceramic acetabular cup components 56, although any other number of cup diameters may be envisioned.

For example, a femoral component 10 having a given size of stem 14 and a 40 mm head diameter may be used with a cup 56 having an outside diameter in the range of 46 mm to 54 mm, although any value within or outside of the range may be envisioned. The 44 mm head 12 may be used with a cup 56 having an outside diameter in the range of 50 mm to 58 mm, although any value within or outside of the range may be envisioned.

Furthermore, each cup 56 may be usable with at least two femoral components 10 having different sizes, and preferably at least three different sizes. The different sizes may be due to having a different-sized stem 14 but same-sized head 12, a same-sized stem 14 but a different-sized head 12, or both different-sized heads 12 and stems 14.

For example, a cup 56 with a 50 mm diameter may be used with a femoral component 10 having a 40 mm head 12 and either Size Three or a Size Four stem 14. Additionally, the same cup 56 may be used with a femoral component 10 having a Size Five stem 14 and either a 40 mm or a 44 mm head 12 diameter.

The femoral component 10, or at least a part thereof, further comprises coating 60a, although this may be optional. The coating 60a is shown in Figures 1 and 2 as a whitened portion of the body 36, although any part of or all of the body and/or stem may comprise coating. Additionally or alternatively, the acetabular cup 56 or part thereof may also comprise coating 60b. Here, an outer surface 62 of the acetabular cup 56 comprises coating 60a. Said coating 60a, 60b improves the engagement with the bone, by increasing the friction therebetween and/or by encouraging bone growth therethrough, therein, thereagainst or therearound, to improve bonding between the femoral bone and the femoral component 10 and/or acetabular cup 56. The coating may also have antibacterial properties. Said coating 60a, 60b is a plasma coating of Hydroxyapatite overlaid over titanium plasma, but any other suitable alternative may be envisioned, such as but not limited to porous ceramic.

In use, a user, such as a surgeon, would select and retrieve from an inventory a femoral component 10 which is appropriately-sized to the patient to be fitted with the femoral component 10. The inventory to be sourced and stocked is much reduced due to the ability to use each femoral component 10 with a range of cups 56 or acetabulum socket sizes. If the acetabulum socket is sufficiently healthy, there may be no need to fit an acetabular cup such that the surgery is hemiarthroplasty. However, in the case of total hip arthroplasty, the total hip replacement system 58 comprising both femoral component 10 and acetabular cup 56 is retrieved.

The patient may be scanned, for example via CT or MRI, and the hip anatomy measured to inform which size of femoral component 10 and/or cup 56 is most appropriate. In particular, the femoral implant 10 is based on the size of the femur head 12. The femoral prosthesis or implant 10 may also be selected based on the size of the cup 56.

Prior to selection of the hip prosthetic system 58 and/or the femoral component 10, the ceramic component or components may need to be manufactured. This may be done on a wide scale for cost reduction or on individual cases, to create customised implants.

Zirconium Oxide, preferably in powder form and/or toughened, and Aluminium Oxide are obtained in sufficient quantities to form the desired prosthetic component. The powders are mixed according to the desired proportions to form a homogenous mixture in powder form. Here, the Zirconium Oxide toughened with Strontium Oxide is 20% w/w and the Aluminium Oxide is 80% w/w, at least in the final product.

Any of the different techniques well known to the person skilled in the art may be used to process the ceramic material in powder form comprising Alumina and/or Zirconia, preferably toughened, into the final product and/or the green compact. Preferably, the powder or powder mixture is inserted into a mould or cavity shaped to form the femoral implant and/or cup.

The techniques may include die pressing, such as cold but preferably here, hot pressing. Isostatic pressing, whether hot or cold, may involve applying pressure in multiple directions through a gaseous or liquid medium surrounding the powder composite, which may be received within a mould shaped to form the green compact of the acetabular cup 56 and/or the femoral component 10. Injection moulding involves mixing the ceramic powder with a binder, such as a low melt polymer, injecting the mixture into a mould, and after cooling, removal of the binder such as via thermal debinding or solvent debinding to form the green compact. Extrusion, slip casting, or gel casting may be alternative options. In each case, the green compact is sintered, or heated to a high temperature, to form the final product. Typically, the green compact is heated to a temperature between 800 and 1500°C, and more preferably to about 1000°C.

The surface roughness of the cup 56 and/or femoral components 10 or parts thereof may be further reduced by grinding and/or polishing, if desired.

The coating 60 is applied to the hip prosthesis or part thereof, although this step may be optional.

A surgical procedure, in this case a hip replacement surgery, may be performed to apply the femoral component 10 and/or the acetabular cup 56 in vivo, to the patient. The surgery involves making an incision adjacent to the relevant hip, preferably but not necessarily, posteriorly. The hip joint is exposed and femur dislocated from the acetabulum. The bone is resected to remove the natural femur head, neck, and optionally a portion of the shaft, as required.

The stem 14 of the femoral component 10 is to be at least partly received in a cavity in the resected femur, here the medullary canal. The volume of the cavity may also be further increased and/or the shape of the cavity altered by the surgeon by removal of additional bone, such as by drilling in order to enable the femoral stem 14 to be received therein. The cavity and in particular, at least part of the inner surface thereof may also be roughened, rasped or broached for improved frictional fit with the femoral component.

Bone cement may be applied to the cavity and/or to the surface of the resected femur to improve bonding or fixing of the femoral component with the bone. However, a short-stem 14 femoral component 10, such as the type used here, is typically cementless i.e. used without bone cement as an adhesive. Instead, the coating 60 on the femoral component 10 encourages bone growth around and into the implant 10, and specifically into the body 36 of the stem 14.

The femoral stem 14 of is subsequently inserted, distal tip 44 first into the cavity. Gentle hammering is typically used to avoid or reduce damage to the femoral component 10 whilst fixing the stem body 36 in the cavity. As the femoral components 10 are asymmetric, care must be taken to ensure that a left-leg femoral component 10 is used in the left femur and/or a right-leg femoral component 10 is used in the right femur. The stem body 36 being curved in at least two directions introduces further asymmetry, to prevent or inhibit rotation of the stem 14 within the bone and/or inhibit pull-off removal of the femoral component 10 from the femur. Thus, care must be taken to insert the femoral component 10 in the correct orientation in the bone as subsequent rotation is inhibited and/or undesirable, and damaging.

As the femoral component 10 is a monobloc, it is not possible to change the head 12 size alone independently of the stem 14, once the stem 14 has been inserted into the bone cavity. However, should the wrong head 12 size be selected, it is possible to remove the femoral component 10 and insert another femoral component 10 having a different head 12 size. If the stems 14 are the same size, this avoids the need to clean out and/or reshape the cavity.

This is particularly beneficial if a smaller head 12 size is required as typically the stem would also be smaller, such that the interference fit with the cavity is worse or compromised.

If performing a total hip replacement, the acetabulum socket is typically prepared by removal of cartilage and potentially enlarged to enable an acetabular cup 56 to be received therein. The cup 56 may be secured via an adhesive such as bone cement. An acetabular cup 56 liner may also be used but this may be optional.

The procedure may involve trialling at least one size of cup 56 and/or femoral component 10 before insertion of the final hip component or components. However, the overall method of implantation of the femoral component is simpler as there is no need to trial different-sized heads during surgery and subsequently attach the head and neck.

The femur is re-aligned and the head 12 of the femoral implant is made to engage with the cup 56 and/or the liner therein, if a liner is used or the acetabulum socket if no cup is used.

The wound is cleaned up to reduce the risk of debris and/or foreign bodies being left within, and the incision may be closed up, usually by stitching.

The surgeon may test the range of motions enabled by the joint before and/or after sealing the incision.

The ceramic material and the femoral component 10 being preferably a monobloc reduces the likelihood of revision surgery, by removal of a source of metal ions, and debris. The coating 60 enhances or encourages bone growth therearound, to further improve the engagement of the femoral component 10 and the femur.

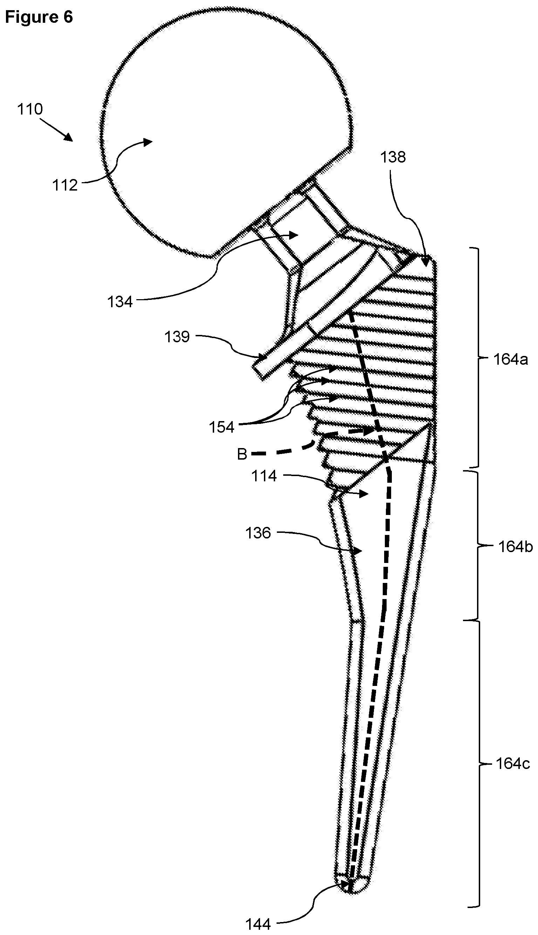

Referring now to Figure 6, there is a shown a second embodiment of a femoral component 110, with a head 112 and a neck 134. Features of the second embodiment which are similar to those of the first embodiment have similar references, with the prefix "1" added.

The femoral component 110 of the second embodiment is similar to the femoral component 10 of the first embodiment, having a head 112, flange, stem 114, neck 134, body 136, distal tip 144, and at least one engagement-enhancing portion 154. Preferably, the femoral component 110 is a monobloc component. Furthermore, the femoral component 110 is formed of the same or similar materials as described in the first embodiment, which are preferably ceramics. Furthermore, the stem 114 comprises a shoulder 138 and a collar 139 formed of part of the neck 134 and/or the body 136, and both in this case, as shown in Figure 6.

The stem 114 extends in this embodiment in or substantially in a plane and/or the distal tip 144 is included in the plane. Preferably said plane is the sagittal plane.

The body 136 preferably has three sub-portions 164a, 164b, 164c which are here integrally formed with each other, although fewer or more than three sub-portions could be envisioned. Whereas the stem body 36 of the first embodiment is curved and/or has a double-taper or a taper in two dimensions, preferably at least part of the body 136 and/or at least one said sub-portion 164a, 164b, 164c of the second embodiment is or is substantially linear and/or non-curved. Here, the body axis B, indicated as dashed lines in Figure 6, is linear or substantially linear in each sub-portion 164a, 164b although either or both may be non-linear in an alternative embodiment.

The body 136 also preferably tapers in one dimension only such that a thickness or width perpendicular to the sagittal plane of the stem 114 is or is substantially constant. The body width may even be similar or the same as the width and/or diameter of the neck 134. In an alternative embodiment, the body 136 and/or stem 114 may taper in more than one plane and/or dimension, for example the body may taper in all three planes, the frontal coronal plane, the transverse plane and/or the sagittal plane. Furthermore, the body may have any desirable cross-sectional shape.

The uses of the second embodiment are the same as the first embodiment. Detailed description is omitted for brevity.

Referring now to Figure 7, there is shown a cross-sectional view of a third embodiment of a femoral component 210. The femoral component 210 of the third embodiment is similar to the femoral component 10 of the first embodiment. Detailed description of the common features is omitted for brevity. Features of the third embodiment which are similar to those of the first embodiment have similar references, with the prefix "2" added. In third embodiment, the collar 239, indicated as dotted lines in Figure 7, does not extend around the entire circumference of the stem 214. Preferably, the collar 239 or a major portion thereof is opposite the shoulder 238, indicated as dashed lines. The uses of the third embodiment are the same as the first embodiment. Detailed description is omitted for brevity.

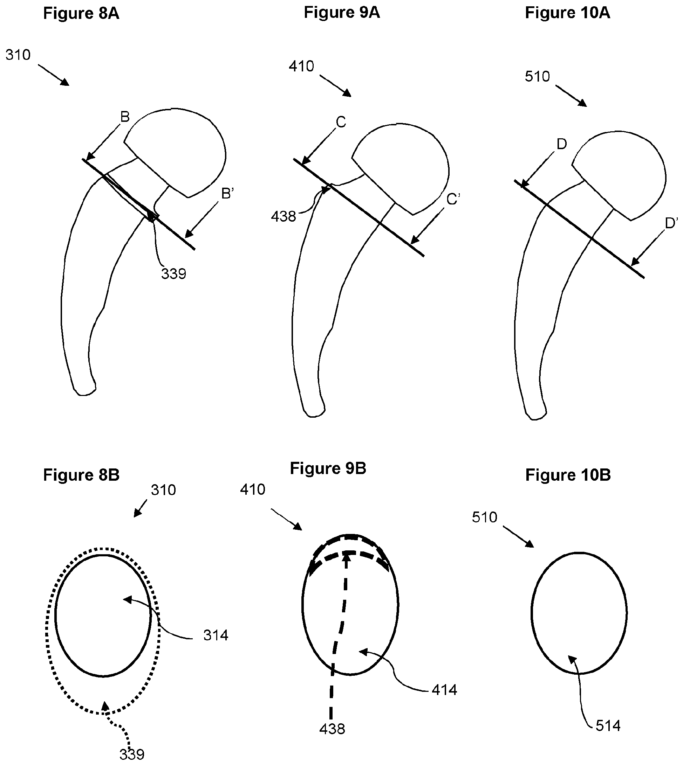

Referring now to Figures 8A and 8B, there is shown a cross-sectional view of a fourth embodiment of a femoral component 310. The femoral component 310 of the fourth embodiment is similar to the femoral component 10 of the first embodiment. Detailed description of the common features is omitted for brevity. Features of the fourth embodiment which are similar to those of the first embodiment have similar references, with the prefix "3" added. In fourth embodiment, the shoulder is omitted such that the femoral component 310 is or is substantially "shoulderless". The collar 339 may or may not extend around the entire circumference of the stem 314. The uses of the fourth embodiment are the same as the first embodiment. Detailed description is omitted for brevity.

Referring now to Figures 9A and 9B, there is shown a cross-sectional view of a fifth embodiment of a femoral component 410, in particular a cross-sectional view of the stem 414 thereof. The femoral component 410 of the fifth embodiment is similar to the femoral component 10 of the first embodiment. Detailed description of the common features, such as the shoulder 438, is omitted for brevity. Features of the fifth embodiment which are similar to those of the first embodiment have similar references, with the prefix "4" added. In fifth embodiment, the collar is omitted such that the femoral component 410 is or is substantially "collarless". The uses of the fifth embodiment are the same as the first embodiment. Detailed description is omitted for brevity.

Referring now to Figures 10A and 10B, there is shown a cross-sectional view of a sixth embodiment of a femoral component 510, in particular a cross-sectional view of the stem 514 thereof. The femoral component 510 of the sixth embodiment is similar to the femoral component 10 of the first embodiment. Detailed description of the common features is omitted for brevity. Features of the sixth embodiment which are similar to those of the first embodiment have similar references, with the prefix "5" added. In sixth embodiment, both the collar and the shoulder are omitted such that the femoral component 510 is or is substantially "collarless" and "shoulderless". The uses of the sixth embodiment are the same as the first embodiment. Detailed description is omitted for brevity.

Whilst preferably the stems 14; 114 in both embodiments have at least one shoulder 38;138, and at least one collar 39;139, it could be envisioned that the shoulder and/or the collar may be omitted in the first and second embodiments. The resulting stem may thus have a neck transitioning substantially more smoothly into the body without forming an edge or protrusion. Such a femoral component may be considered to be "collarless" and/or "shoulderless".

Whilst in this embodiment, the femoral component 10 is designed for use in either the left leg or the right leg, it could easily be envisioned that the femoral component may be symmetric and/or formed so as to be usable with either leg. This would further reduce the inventory required to be sourced and stocked, further reducing costs and storage space.

Although the Alumina, the Zirconia and the Strontium Oxide are preferably provided at 80%, 17% and 3% w/w respectively, alternative ranges may be envisioned. For instance, the Aluminium Oxide may be provided as 15 to 25% w/w, and more preferably as 20% w/w. The Zirconium Oxide may be provided as 70 to 85% w/w. If provided without Alumina, Zirconia and/or toughened Zirconia may be provided at a much higher percentage, such as up to 100% or thereabouts. Whilst in this embodiment, the Strontium Oxide is 3% w/w, the percentage may vary according to the desired properties as lower percentages increase fracture toughness but may reduce mechanical strength and age resistance. It may even be envisioned that the Zirconium Oxide may not necessarily be toughened.

In the present embodiment, the Zirconium Oxide is toughened by Strontium Oxide, but in an alternative embodiment, the Zirconium Oxide may be toughened by any other Oxide or combination of Oxides, such as Magnesium Oxide, Calcium Oxide, Cerium (III) Oxide, Yttrium Oxide which may be Yttrium (III) Oxide, or any other suitable stabilising Oxide.

A suitable alternative ceramic material may have any of the following characteristics: be formed of or substantially of 75% w/w Aluminium Oxide, be formed of or substantially of 25% Yttria-toughened Zirconia, have a density of or approximately of 4.37 g/cm³, a fracture toughness of at least 5 MPa√m, a grain size is or is substantially 0.8 µm, a hardness of 14.7 GPa or 1500HV, and a biaxial bending strength of at least 700 MPa. Another suitable alternative ceramic material may have any of the following characteristics: be formed of or substantially of 20% w/w Aluminium Oxide, be formed of or substantially of 80% Yttria-toughened Zirconia, a density of or of approximately 5.51 g/cm³, a fracture toughness of at least 7 MPa√m, a grain size is or is substantially 0.4 µm, and a biaxial bending strength of at least 900 MPa.

Although preferably formed of only ceramics, it may be envisioned that the cup and/or a cup liner if used may be formed of metal or other materials, whether alone or in combination as a composite. For instance, the cup may be partially or fully formed of at least one of: one or more metals; a composite; an alloy; a non-metallic inclusion; a metallic biomaterial; a metal matrix composite, such as with ceramics reinforcements; and plastics. Said plastics may include polyethylene. Said metal or metals may be hardened and/or be in a form other than in oxide, carbide and/or nitride forms. For example, the cup, or part of may comprise at least one of: cobalt; chrome; molybdenum; titanium; nickel; steel, preferably stainless steel, and any other suitable metal currently used for femoral components, such as the titanium alloy Ti-6AI-4V.

In particular, it may be envisioned that the cup may be a metal - ceramic hybrid or composite. For example, the cup may have a metallic core or backbone, which may or may not be a monobloc. The metal backbone may be partly or fully encased in a ceramics shell, which may be monobloc. The ceramics may even be formed of hydroxyapatite ceramics. The core may provide strength or stiffness, whilst the ceramics material may be more biocompatible and/or may contain or inhibit outward leeching of any metal ions from the core. The method of manufacture may need to be altered according to the properties of the materials. Said properties may include the relative sintering and/or melting temperatures of ceramics and the metal or alloy; their expansion properties; chemical reactions with each other, which may be temperature-dependent; or any other relevant material properties. For example, the core may be formed first, and the ceramics shell formed secondarily therearound where the melting temperature of core exceeds the sintering temperature of the ceramics shell. In the opposite case, it may be envisioned that a hollow ceramics shell may be formed first and molten metal poured, injected or inserted secondarily therein. In this latter case, it may be desirable to provide a plug or sealing element to plug or seal any hole in the shell. Further obvious modifications may be apparent to the person skilled in the art.

Whilst the part-spherical surface 26 is described as being convex, it is appreciated that the surface may in fact not be convex and/or may be at least in part multifaceted and/or planar, if required. Whilst the preferred embodiment is a short-stem 14, particularly shaped to inhibit or prevent rotation relative to the femur, this may not necessarily be the case. For instance, the femoral component may have a long stem and/or be symmetric. The stem or part thereof may be cylindrical, or any other cross-section such as an oval or egg-shaped, ellipse, tear shaped, partly curved, partly linear, and polygonal. The cross-section may not necessarily vary with increasing distance from the head.

In the present embodiment, the flange 20 is planar and said plane is normal to the polar axis. It could however be envisioned that the plane may not be normal to the polar axis, and/or may be normal to another axis, such as the body axis. It may alternatively be envisioned that the flange is not planar. For instance, the flange may be curved or non-curved, part of a cone, polygonal or any other suitable or desirable shape in latitudinal or longitudinal cross-section.

The flange may even have one or more protrusions and/or recesses or grooves along at least part of the circumferential or longitudinal extent of the flange and/or along the medial or lateral extent of the flange. Protrusions and/or recesses such as crenellations or castellations, may further prevent or inhibit rotation of the femoral implant. Whilst the neck 34 transitions smoothly into the head, it could be envisioned that at least one edge is formed between the flange and the neck. This may occur, for instance, where the neck meets the flange and/or planar undersurface at substantially a right angle in longitudinal cross-section. It may also be envisioned that the neck widens outwardly adjacent to the planar undersurface. At least one further surface or plane may meet the neck and/or the planar undersurface. The angle formed in longitudinal cross-section with each may be substantially 45°, although any other combination of angles adding up to a right angle, such as 30°/60° may be envisioned.

It may even be envisioned that the flange may be partly or entirely omitted. In this alternative embodiment, the femoral head may not be truncated such that the femoral head and/or the articulating surface may be or be substantially spherical and/or the articulating surface may meet with the neck.

Whilst the taper of the body is monotonically decreasing with increasing distance from the head 12, it could be envisioned that the body may not be monotonically decreasing. For example, the taper may increase before decreasing, with increasing distance from the head. Alternatively, there may be no tapering such that the thickness of the stem is substantially uniform throughout, barring any grooves or other grip-enhancing features.

Preferably, the whole cup 56 is formed of ceramic but it may be envisioned that only part of the acetabular cup may be formed of ceramic. For example, at least part of the outer and/or inner surface thereof, may be ceramic.

It is therefore possible to provide a femoral component which is formed of a strong and substantially biocompatible ceramic material. Being integrally formed improves the structural integrity of the implant, whilst reducing the likelihood of debris. It is also possible to provide a kit of multiple femoral components, having same dimensioned stems and a range of head sizes for keeping constant the amount of bone to be removed, regardless of the head size. It is therefore also possible to provide a kit comprising a plurality of different-sized femoral implants, and a system comprising a femoral implant and an acetabular component for use in surgery. Both cup and femoral implant being formed of ceramic removes the need to use metal. It is also possible to provide a method of manufacture and of use which are simplified by virtue of not requiring head and stem components and trialling of heads.

The words 'comprises/comprising' and the words 'having/including' when used herein with reference to the present invention are used to specify the presence of stated features, integers, steps or components, but do not preclude the presence or addition of one or more other features, integers, steps, components or groups thereof. It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination. The embodiments described above are provided by way of examples only, and various other modifications will be apparent to persons skilled in the field without departing from the scope of the invention as defined by the claims.

## Claims

1. A ceramic monobloc femoral component (10; 110; 210; 310; 410; 510)having a ceramic femoral stem (14; 114; 214; 314; 414; 514) and a ceramic head (12; 112) defining a part-spherical articular surface receivable by an acetabular cup (56), the ceramic femoral stem (14; 114; 214; 314; 414; 514) and the ceramic head (12; 112) being integrally formed as one-piece, wherein the ceramic monobloc femoral component (10; 110; 210; 310; 410; 510) is formed of a ceramic material which includes a percentage of Aluminium Oxide and a percentage of Zirconium Oxide, **characterised in that** the percentage of Aluminium Oxide is in a range of or substantially of 15% to 85% by weight and wherein the percentage of Zirconium Oxide is in a range of or substantially of 15% to 85% by weight and that the ceramic monobloc femoral component (10; 110; 210; 310; 410; 510) is configured for a total hip replacement prosthesis involving complete replacement of a femoral head.

2. A ceramic monobloc femoral component (10; 110; 210; 310) as claimed in claim 1, the ceramic femoral stem (14; 114; 214; 314) further comprising a collar (39; 139; 239; 339) terminating between a tangential plane (50) of an equatorial circumference (22) of the ceramic head (12; 112) and a polar-axial plane (52) parallel to said tangential plane (50).

3. A ceramic monobloc femoral component (10; 110; 210; 310; 410; 510) as claimed in claim 1 or in claim 2, wherein the head (12; 112) is retroverted relative to the sagittal plane (45) of the ceramic monobloc femoral component (10; 110; 210; 310; 410; 510).

4. A ceramic monobloc femoral component (10; 110; 210; 310; 410; 510) as claimed in any one of the preceding claims, wherein the percentage of Aluminium Oxide is or is substantially 70% to 85% by weight.

5. A ceramic monobloc femoral component (10; 110; 210; 310; 410; 510) as claimed in any one of claims 1 to 3, wherein the percentage of Aluminium Oxide is or is substantially 15% to 25% by weight.

6. A ceramic monobloc femoral component (10; 110; 210; 310; 410; 510) as claimed in any one of the preceding claims, wherein the percentage of Zirconium Oxide is or is substantially 15% to 25% by weight.

7. A ceramic monobloc femoral component (10; 110; 210; 310; 410; 510) as claimed in any one of claims 1 to 3 or claim 5 , wherein the percentage of Zirconium Oxide is or is substantially 70% to 85% by weight.

8. A ceramic monobloc femoral component (10; 110; 210; 310; 410; 510) as claimed in any one of the preceding claims, wherein the Zirconium Oxide is toughened Zirconium Oxide which is toughened by Strontium Oxide and/or Yttrium Oxide.

9. A ceramic monobloc femoral component (10; 110; 210; 310; 410; 510) as claimed in any of the preceding claims, wherein the ceramic head (12; 112) further comprises a flange (20) having a medial extent (32) which is equivalent to or greater than half a radius (40) of a neck (34) of the ceramic femoral stem (14; 114; 214; 314; 414; 514).

10. A ceramic monobloc femoral component (10; 110; 210; 410) as claimed in any of the preceding claims, the ceramic femoral stem (14; 114; 214; 414) further comprising a shoulder (38; 138; 238; 438) terminating between a tangential plane (50) of an equatorial circumference (22) of the ceramic head (12; 112) and a polar-axial plane (52) parallel to said tangential plane (50).

11. A ceramic monobloc femoral component kit comprising a plurality of ceramic monobloc femoral components (10; 110; 210; 310; 410; 510) as claimed in any preceding claim, a first said ceramic monobloc femoral component (10; 110; 210; 310; 410; 510) having a said femoral stem (14; 114; 214; 314; 414; 514) and a said femoral head (12; 112), and a second said femoral component (10; 110; 210; 310; 410; 510) having a said femoral stem (14; 114; 214; 314; 414; 514) and a said femoral head (12; 112), wherein the said femoral stems (14; 114; 214; 314; 414; 514) are same-dimensioned and the femoral heads (12; 112) are different-dimensioned.

12. A total hip replacement system (58) comprising at least one ceramic monobloc femoral component (10; 110; 210; 310; 410; 510) as claimed in any of claims 1 to 10, and at least one ceramic acetabular cup component (56) for receiving the ceramic head (12; 112) of the ceramic monobloc femoral component (10; 110; 210; 310; 410; 510).

13. A total hip replacement system (58) as claimed in claim 12, wherein the total hip replacement system (58) comprises a plurality of ceramic monobloc femoral components (10; 110; 210; 310; 410; 510) and a plurality of ceramic acetabular cup components (56), each said ceramic monobloc femoral component (10; 110; 210; 310; 410; 510) being matched with at least a pair of dimensionally different said ceramic acetabular cup components (56).

14. A total hip replacement system (58) as claimed in claim 13, wherein a said ceramic monobloc femoral component (10; 110; 210; 310; 410; 510) is matched with at least three dimensionally different said ceramic acetabular cup components (56).

15. A total hip replacement system (58) as claimed in any of claims 12 to 14, wherein at least one of the femoral components (10; 110; 210; 310; 410; 510) and the acetabular cup (56) is or is substantially free from metal other than in oxide, carbide or nitride forms.

## Patentansprüche

1. Keramische Monoblock-Femurkomponente (10; 110; 210; 310; 410; 510), aufweisend einen keramischen Femurschaft (14; 114; 214; 314; 414; 514) und einen keramischen Kopf (12; 112), der eine teilsphärische Gelenkfläche definiert, die durch eine Hüftgelenkspfanne (56) aufgenommen werden kann, wobei der keramische Femurschaft (14; 114; 214; 314; 414; 514) und der keramische Kopf (12; 112) integral als ein Stück hergestellt sind, wobei die keramische Monoblock-Femurkomponente (10; 110; 210; 310; 410; 510) aus einem Keramikmaterial hergestellt ist, das einen Prozentanteil von Aluminiumoxid und einen Prozentanteil von Zirkonoxid einschließt, **dadurch gekennzeichnet, dass** der Prozentanteil von Aluminiumoxid in einem Bereich von im Wesentlichen 15 Gew.-% bis 85 Gew.-% liegt, und wobei der Prozentanteil von Zirkonoxid in einem Bereich von im Wesentlichen 15 Gew.-% bis 85 Gew.-% liegt, und dass die keramische Monoblock-Femurkomponente (10; 110; 210; 310; 410; 510) für eine Hüfttotalprothese konfiguriert ist, die einen Komplettersatz eines Femurkopfs beinhaltet.

2. Keramische Monoblock-Femurkomponente (10; 110; 210; 310) nach Anspruch 1, wobei der keramische Femurschaft (14; 114; 214; 314) ferner einen Kragen (39; 139; 239; 339) umfasst, der zwischen einer Tangentialebene (50) eines Äquatorialumfangs (22) des keramischen Kopfs (12; 112) und einer polar-axialen Ebene (52) endet, die parallel zu der besagten Tangentialebene (50) ist.

3. Keramische Monoblock-Femurkomponente (10; 110; 210; 310; 410; 510) nach Anspruch 1 oder Anspruch 2, wobei der Kopf (12; 112) in Bezug auf die Sagittalebene (45) der keramischen Monoblock-Femurkomponente (10; 110; 210; 310; 410; 510) retrovertiert ist.

4. Keramische Monoblock-Femurkomponente (10; 110; 210; 310; 410; 510) nach einem der vorhergehenden Ansprüche, wobei der Prozentanteil von Aluminiumoxid 70 Gew.-% bis 85 Gew.-% beträgt oder im Wesentlichen 70 Gew.-% bis 85 Gew.-% beträgt.

5. Keramische Monoblock-Femurkomponente (10; 110; 210; 310; 410; 510) nach einem der Ansprüche 1 bis 3, wobei der Prozentanteil von Aluminiumoxid 15 Gew.-% bis 25 Gew.-% beträgt oder im Wesentlichen 15 Gew.-% bis 25 Gew.-% beträgt.

6. Keramische Monoblock-Femurkomponente (10; 110; 210; 310; 410; 510) nach einem der vorhergehenden Ansprüche, wobei der Prozentanteil von Zirkonoxid 15 Gew.-% bis 25 Gew.-% beträgt oder im Wesentlichen 15 Gew.-% bis 25 Gew.-% beträgt.

7. Keramische Monoblock-Femurkomponente (10; 110; 210; 310; 410; 510) nach einem der Ansprüche 1 bis 3 oder Anspruch 5, wobei der Prozentanteil von Zirkonoxid 70 Gew.-% bis 85 Gew.-% beträgt oder im Wesentlichen 70 Gew.-% bis 85 Gew.-% beträgt.

8. Keramische Monoblock-Femurkomponente (10; 110; 210; 310; 410; 510) nach einem der vorhergehenden Ansprüche, wobei das Zirkonoxid verstärktes Zirkonoxid ist, das durch Strontiumoxid und/oder Yttriumoxid verstärkt ist.

9. Keramische Monoblock-Femurkomponente (10; 110; 210; 310; 410; 510) nach einem der vorhergehenden Ansprüche, wobei der keramische Kopf (12; 112) ferner einen Flansch (20) umfasst, der eine mediale Erstreckung (32) aufweist, die äquivalent zu einer oder größer als eine Hälfte eines Radius (40) eines Halses (34) des keramischen Femurschafts (14; 114; 214; 314; 414; 514) ist.

10. Keramische Monoblock-Femurkomponente (10; 110; 210; 410) nach einem der vorhergehenden Ansprüche, wobei der keramische Femurschaft (14; 114; 214; 414) ferner eine Schulter (38; 138; 238; 438) umfasst, die zwischen einer Tangentialebene (50) eines Äquatorialumfangs (22) des keramischen Kopfs (12; 112) und einer polar-axialen Ebene (52) endet, die parallel zu der besagten Tangentialebene (50) ist.

11. Kit von keramischen Monoblock-Femurkomponenten, umfassend eine Vielzahl von keramischen Monoblock-Femurkomponenten (10; 110; 210; 310; 410; 510) nach einem vorhergehenden Anspruch, wobei eine erste besagte keramische Monoblock-Femurkomponente (10; 110; 210; 310; 410; 510) einen besagten Femurschaft (14; 114; 214; 314; 414; 514) und einen besagten Femurkopf (12; 112) aufweist und eine zweite besagte Femurkomponente (10; 110; 210; 310; 410; 510) einen besagten Femurschaft (14; 114; 214; 314; 414; 514) und einen besagten Femurkopf (12; 112) aufweist, wobei die besagten Femurschäfte (14; 114; 214; 314; 414; 514) gleich dimensioniert sind und die Femurköpfe (12; 112) unterschiedlich dimensioniert sind.

12. Hüfttotalersatzsystem (58), umfassend mindestens eine keramische Monoblock-Femurkomponente (10; 110; 210; 310; 410; 510) nach einem der Ansprüche 1 bis 10 und mindestens eine keramische Hüftgelenkspfannenkomponente (56) zum Aufnehmen des keramischen Kopfs (12; 112) der keramischen Monoblock-Femurkomponente (10; 110; 210; 310; 410; 510).

13. Hüfttotalersatzsystem (58) nach Anspruch 12, wobei das Hüfttotalersatzsystem (58) eine Vielzahl von keramischen Monoblock-Femurkomponenten (10; 110; 210; 310; 410; 510) und eine Vielzahl von keramischen Hüftgelenkspfannenkomponenten (56) umfasst, wobei jede besagte keramische Monoblock-Femurkomponente (10; 110; 210; 310; 410; 510) mit mindestens einem Paar von dimensional unterschiedlichen besagten keramischen Hüftgelenkspfannenkomponenten (56) abgeglichen ist.

14. Hüfttotalersatzsystem (58) nach Anspruch 13, wobei eine besagte keramische Monoblock-Femurkomponente (10; 110; 210; 310; 410; 510) mit mindestens drei dimensional unterschiedlichen besagten keramischen Hüftgelenkspfannenkomponenten (56) abgeglichen ist.

15. Hüfttotalersatzsystem (58) nach einem der Ansprüche 12 bis 14, wobei mindestens eine der Femurkomponenten (10; 110; 210; 310; 410; 510) und die Hüftgelenkspfanne (56) im Wesentlichen frei von Metall mit Ausnahme von in Oxid-, Karbid- oder Nitridformen sind.

## Revendications

1. Un composant fémoral monobloc en céramique (10 ; 110 ; 210 ; 310 ; 410 ; 510) comportant une tige fémorale en céramique (14 ; 114 ; 214 ; 314 ; 414 ; 514) et une tête en céramique (12 ; 112) définissant une surface articulaire partiellement sphérique pouvant être logée dans une cupule acétabulaire (56), ladite tige fémorale en céramique (14 ; 114 ; 214 ; 314 ; 414 ; 514) et la tête en céramique (12 ; 112) étant formées d'un seul tenant en une pièce monobloc, ledit composant fémoral monobloc en céramique (10 ; 110 ; 210 ; 310 ; 410 ; 510) étant constitué d'un matériau céramique qui comprend un pourcentage d'oxyde d'aluminium et un pourcentage d'oxyde de zirconium, **caractérisé en ce que** le pourcentage d'oxyde d'aluminium se situe dans une plage de, ou est sensiblement de, 15 % à 85 % en poids et dans lequel le pourcentage d'oxyde de zirconium se situe dans une plage de, ou est sensiblement de, 15 % à 85 % en poids et **en ce que** ledit composant fémoral monobloc en céramique (10; 110; 210; 310; 410; 510) est configuré pour une prothèse de remplacement total de la hanche impliquant le remplacement complet d'une tête fémorale.

2. Un composant fémoral monobloc en céramique (10 ; 110 ; 210 ; 310) selon la revendication 1, ladite tige fémorale en céramique (14 ; 114 ; 214 ; 314) comprenant en outre une collerette (39 ; 139 ; 239 ; 339) se terminant entre un plan tangentiel (50) d'une circonférence équatoriale (22) de ladite tête en céramique (12 ; 112) et un plan polaire axial (52) parallèle audit plan tangentiel (50).

3. Un composant fémoral monobloc en céramique (10 ; 110 ; 210 ; 310 ; 410 ; 510) selon la revendication 1 ou la revendication 2, dans lequel ladite tête (12 ; 112) est en rétroversion par rapport au plan sagittal (45) du composant fémoral monobloc en céramique (10 ; 110 ; 210 ; 310 ; 410 ; 510).

4. Un composant fémoral monobloc en céramique (10 ; 110 ; 210 ; 310 ; 410 ; 510) selon l'une quelconque des revendications précédentes, dans lequel le pourcentage d'oxyde d'aluminium est ou est sensiblement de 70 % à 85 % en poids.

5. Un composant fémoral monobloc en céramique (10 ; 110 ; 210 ; 310 ; 410 ; 510) selon l'une quelconque des revendications 1 à 3, dans lequel le pourcentage d'oxyde d'aluminium est ou est sensiblement de 15 % à 25 % en poids.

6. Un composant fémoral monobloc en céramique (10 ; 110 ; 210 ; 310 ; 410 ; 510) selon l'une quelconque des revendications précédentes, dans lequel le pourcentage d'oxyde de zirconium est ou est sensiblement de 15 % à 25 % en poids.

7. Un composant fémoral monobloc en céramique (10 ; 110 ; 210 ; 310 ; 410 ; 510) selon l'une quelconque des revendications 1 à 3 ou la revendication 5, dans lequel le pourcentage d'oxyde de zirconium est ou est sensiblement 70 % à 85 % en poids.

8. Un composant fémoral monobloc en céramique (10 ; 110 ; 210 ; 310 ; 410 ; 510) selon l'une quelconque des revendications précédentes, dans lequel l'oxyde de zirconium est un oxyde de zirconium renforcé, lequel est renforcé par de l'oxyde de strontium et/ou de l'oxyde d'yttrium.

9. Un composant fémoral monobloc en céramique (10 ; 110 ; 210 ; 310 ; 410 ; 510) selon l'une quelconque des revendications précédentes, dans lequel la tête en céramique (12 ; 112) comprend en outre une ailette (20) présentant une étendue médiale (32) qui est supérieure ou égale à la moitié d'un rayon (40) d'un col (34) de la tige fémorale en céramique (14 ; 114 ; 214 ; 314 ; 414 ; 514).

10. Un composant fémoral monobloc en céramique (10 ; 110 ; 210 ; 410) selon l'une quelconque des revendications précédentes, la tige fémorale en céramique (14 ; 114 ; 214 ; 414) comprenant en outre un épaulement (38 ; 138 ; 238 ; 438) se terminant entre un plan tangentiel (50) d'une circonférence équatoriale (22) de ladite tête en céramique (12 ; 112) et un plan polaire axial (52) parallèle audit plan tangentiel (50).

11. Un kit de composants fémoraux monoblocs en céramique comprenant une pluralité de composants fémoraux monoblocs en céramique (10 ; 110 ; 210 ; 310 ; 410 ; 510) selon l'une quelconque des revendications précédentes, un premier desdits composants fémoraux monoblocs en céramique (10 ; 110 ; 210 ; 310 ; 410 ; 510) ayant une dite tige fémorale (14 ; 114 ; 214 ; 314 ; 414 ; 514) et une dite tête fémorale (12 ; 112), et un second desdits composants fémoraux (10 ; 110 ; 210 ; 310 ; 410 ; 510) ayant une dite tige fémorale (14 ; 114 ; 214 ; 314 ; 414 ; 514) et une dite tête fémorale (12 ; 112), dans lequel lesdites tiges fémorales (14 ; 114 ; 214 ; 314 ; 414 ; 514) sont dimensionnellement identiques et les têtes fémorales (12 ; 112) sont dimensionnellement différentes.

12. Un système de remplacement total de la hanche (58) comprenant au moins un composant fémoral monobloc en céramique (10 ; 110 ; 210 ; 310 ; 410 ; 510) selon l'une quelconque des revendications 1 à 10, et au moins un composant de cupule acétabulaire en céramique (56) destiné à recevoir la tête en céramique (12 ; 112) dudit composant fémoral monobloc en céramique (10 ; 110 ; 210 ; 310 ; 410 ; 510).

13. Un système de remplacement total de la hanche (58) selon la revendication 12, dans lequel ledit système de remplacement total de la hanche (58) comprend une pluralité de composants fémoraux monoblocs en céramique (10 ; 110 ; 210 ; 310 ; 410 ; 510) et une pluralité de composants de cupule acétabulaire en céramique (56), chaque dit composant fémoral monobloc en céramique (10 ; 110 ; 210 ; 310 ; 410 ; 510) étant apparié avec au moins une paire desdits composants de cupule acétabulaire en céramique (56) dimensionnellement différents.

14. Un système de remplacement total de la hanche (58) selon la revendication 13, dans lequel un dit composant fémoral monobloc en céramique (10 ; 110 ; 210 ; 310 ; 410 ; 510) est apparié avec au moins trois dits composants de cupule acétabulaire en céramique (56) dimensionnellement différents.

15. Un système de remplacement total de la hanche (58) selon l'une quelconque des revendications 12 à 14, dans lequel au moins l'un des composants fémoraux (10 ; 110 ; 210 ; 310 ; 410 ; 510) et la cupule acétabulaire (56) est ou est sensiblement exempt de métal autre que sous forme d'oxyde, de carbure ou de nitrure.
